**Europäisches Patentamt**

**European Patent Office**

**Office européen des brevets**

(11) Veröffentlichungsnummer : **0 325 557 B1**

(12) **EUROPÄISCHE PATENTSCHRIFT**

(51) Int. Cl.⁵ : **A61F 2/32**

(45) Veröffentlichungstag der Patentschrift :
**02.09.92 Patentblatt 92/36**

(21) Anmeldenummer : **89730015.8**

(22) Anmeldetag : **23.01.89**

(30) Priorität : **22.01.88 DE 3802239**

(43) Veröffentlichungstag der Anmeldung :
**26.07.89 Patentblatt 89/30**

(45) Bekanntmachung des Hinweises auf die
Patenterteilung :
**02.09.92 Patentblatt 92/36**

(84) Benannte Vertragsstaaten :
**AT BE CH DE ES FR GB GR IT LI NL SE**

(56) Entgegenhaltungen :
**EP-A- 0 181 586**
**EP-A- 0 255 797**
**DE-A- 3 120 147**

(73) Patentinhaber : **Goymann, Volkmar, Prof. Dr.
med.**
**Forstmannstrasse 56**
**W-4300 Essen 16 (DE)**
Patentinhaber : **Kranz, Curt, Dr.-Ing.**
**Kufsteiner Strasse 12**
**W-1000 Berlin 62 (DE)**

(72) Erfinder : **Goymann, Volkmar, Prof. Dr. med.**
**Forstmannstrasse 56**
**W-4300 Essen 16 (DE)**
Erfinder : **Anapliotis, Emmanuel**
**Kiebitzweg 5**
**W-1000 Berlin 33 (DE)**
Erfinder : **Kranz, Curt, Dipl.-Ing.**
**Kufsteiner Strasse 12**
**W-1000 Berlin 62 (DE)**

(74) Vertreter : **Christiansen, Henning, Dipl.-Ing.**
**Patentanwalt CHRISTIANSEN Pacelliallee**
**43/45**
**W-1000 Berlin 33 (DE)**

(54) **Endoprothese.**

## Beschreibung

Die Erfindung betrifft eine Schaftendoprothese der im Oberbegriff des Anspruchs 1 angegebenen Art sowie ein Verfahren zur Herstellung einer solchen Endoprothese.

Es sind Hüftgelenkendoprothesen bekannt, die aus einem Prothesenkopf, Prothesenkragen und einem Prothesenschaft bestehen, der im Markraum des Femur eines Patienten unter Verwendung von Knochenzement oder zementlos verankert wird. Bei zementloser Verankerung des Prothesenschaftes im Markraum des Femur wird eine Schaftstruktur verwendet, die eine möglichst feste Verankerung im Markraum sicherstellt, wobei eine poröse Oberfläche das Einwachsen von spongiösem Material erleichtern und damit die Festigkeit der Verankerung des Prothesenschaftes erhöhen soll. Zur Verankerung des Prothesenschaftes im Markraum ist jedoch eine weitgehende Entfernung der Spongiosa erforderlich. Die Entfernung der Spongiosa ist aber als unphysiologische lokale Störung im Sinne einer Defektbildung anzusehen, da dadurch die Belastungs- und vor allem altersabhängigen Anspassungsmöglichkeiten beeinträchtigt werden.

Eine aus der EP-A-0181586 bekannte massive, einstückige, eintreibbare Hüftgelenkendoprothese weist am proximalen Ende eine Mehrzahl von Rippen auf, die vom Schaftkern sich nach außen erstrecken und deren Rückenflächen gezahnt sind. Die einzelnen Rippen sind derart ausgerichtet, daß sie rechtwinklig oder auch winkelig zueinander stehen. Nachteilig bei dieser bekannten Hüftgelenkprothese ist aber wiederum, daß die in den Körper einzuschlagende Materialmenge nicht gering ist und daß eine nicht unerhebliche Menge an Spongiosa vor der Einbringung der Endoprothese entfernt werden muß.

Ein weiterer Nachteil bekannter Hüftgelenkendoprothesen ist eine ungleichmäßige Kraftverteilung durch eine primär distale Einleitung der Kräfte in den distal der regio intertrochanterica gelegenen Oberschenkelanteil, wodurch der eigentlich dafür vorgesehene Knochenanteil aus der funktionellen Belastung genommen wird. Dies kann zu einer Atrophie führen, wobei der erwünschte funtionelle Reiz für den Knochen, fixierend an die Endoprothese heranzuwachsen, ausbleibt.

Die Folge ist eine vorzeitige Lockerung der Endoprothese mit der Notwendigkeit einer Revisionsoperation. Eine solche Revisionsoperation stößt aber bei vielen Endoprothesen auf erhebliche Schwierigkeiten, da die Prothesenschäfte vieler Endoprothesen so geformt sind, daß ein Entfernen der Endoprothese nur mit einer erheblichen Zerstörung des Knochenmaterials möglich ist, was letztlich das Einwachsen einer neuen Endoprothese erschwert und zum Teil unmöglich macht.

Ein weiterer Nachteil bekannter Hüftgelenkendoprothesen besteht darin, daß zwar Standardmodelle für unterschiedliche Femurgrößen vorhanden sind, diese aber nicht an die individuelle Form des proximalen Femur eines Patienten angepaßt sind, so daß die ungünstige Krafteinleitung vieler Endoprothesen noch verstärkt und die vorhandene Knochensubstanz nicht optimal genutzt wird.

Schließlich machen die bekannten Hüftgelenkendoprothesen eine aufwendige Operationstechnik erforderlich, bei der es notwendig ist, den mit der Prothese zu versehenden Knochen zur Aufnahme des Prothesenschaftes vorzumeißeln oder vorzubohren. Dies muß mit größter Vorsicht geschehen, da weder die Knochenstruktur unnötig beschädigt werden noch eine zu große Aushöhlung geschaffen werden darf, in der der Prothesenschaft nicht ausreichend fest verankert werden kann. Außerdem ist eine erhebliche Zeitspanne zum Raspeln oder Aufbohren erforderlich, was das Infektionsrisiko erheblich erhöht. Schließlich ist die Eröffnung des Markkanals erforderlich und eine erhebliche Gefahr einer Zerstörung des Knochens infolge einer via falsa gegeben.

Der Erfindung liegt die Aufgabe zugrunde, eine Schaftendoprothese der eingangs genannten Art zu schaffen, die die Erhaltung der Physiologie des Knochenmarkraumes einschließlich der intertrochantären Spongiosa und damit günstige anatomische Bedingungen bei Revisionsoperationen und optimale Anpassungsmöglichkeiten an altersbedingte Knochenveränderungen, eine vereinfachte Operationstechnik und eine optimale physiologische Krafteinleitung sicherstellt sowie eine vergrößerte Anlagefläche für das Anwachsen von Knochenmaterial und eine individuelle Anpassung an die Form des Femur eines Patienten ermöglicht. Diese Aufgabe wird mit dem kennzeichnenden Merkmal des Anspruchs 1 gelöst.

Die erfindungsgemäße Lösung ermöglicht das Anbringen einer Endoprothese unter Erhalt der spongiösen Strukturen, so daß die Stoffwechselbiologie nicht gestört und die Durchblutungsbedingungen nicht grundsätzlich geändert werden. Durch den Erhalt der Physiologie des Knochenmarkraumes einschließlich der intertrochantären Spongiosa werden günstige anatomische Bedingungen bei Revisionsoperationen und optimale Anpassungsmöglichkeiten an altersbedingte Knochenveränderungen geschaffen. Die erfindungsgemäße Endoprothese ist mit einem besonders stabilen Schaft versehen, der aus sich in Schaftrichtung erstreckenden Plattenförmigen Lamellen besteht, von denen mindestens zwei bezüglich einer zur Schaftrichtung senkrechten Ebene rechtwinklig aufeinanderstehen, so daß sich eine besondere Festigkeit des Prothesensitzes durch die Blockierung in unterschiedlichen Raumrichtungen ergibt. Die verschieden gerichteten Lamellenteile weisen eine größeres Steifigkeit auf, die aus der unterschiedlichen räumlichen Ausrichtung resultiert.

EP 0 325 557 B1

Einer vorteilhaften Weiterbildung der Erfindung gemäß besteht der Schaft aus Winkelprofilen bzw. aus vorzugsweise rechtwinklig miteinander verbundenen Plattenförmigen Lamellen.

Diese verbiegungssicheren Schaftteile sind, entsprechend einer besonders vorteilhaften Weiterbildung der Erfindung, mit einer peripheren Schneide ausgestattet, wodurch ihre Verankerung in der Spongiosa durch selbstschneidendes Eintreiben erfolgen kann und folglich kein Vorschneiden oder Raspeln erforderlich ist. Wesentliche Voraussetzung für das Eintreiben des Prothesenschaftes ist, daß alle einzutreibenden Plattenförmigen Lamellen des Systems der Endoprothese parallel zur Längsachse des Prothesenschafts gerichtet sind. Die periphere Schneide kann je nach Art der Endoprothese beispielsweise an der einem Kragen oder einer Knochenauflagefläche der Endoprothese abgewandten Kante (z.B. Hüftgelenkendoprothese) des Schaftes vorgesehen sein. Als Schneidkanten sind immer die Kanten in Eintreibungsrichtung ausgeführt.

Dieses Vorgehen bietet entscheidende operationstechnische Vorteile, da ein Raspeln oder Aufbohren nicht notwendig ist, was eine erhebliche Zeitersparnis und eine Minderung des Infektionsrisikos sowie die Vermeidung einer via falsa zur Folge hat.

Da die Anzahl und die Querschnittsform der aus Plattenförmigen Lamellen ausgebildeten Schafteilen beliebig sein kann, wird zum einen eine vergrößerte Anlagefläche für das Anwachsen von Knochenmaterial und zum Anderen die Voraussetzung für eine individuelle Anpassbarkeit der Endoprothese an die jeweilige Knochenform mit geringen Mitteln ermöglicht.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figuren 1 bis 17 verschiedene Ausführungsformen einer intertrochantären Lamellenprothese mit im Querschnitt offener Kontur in Seitenansicht, in perspektivischer Ansicht und z.T. im Querschnitt, sowie

Figuren 18 bis 20 eine intertrochantäre Lamellenprothese mit im Querschnitt geschlossener Kontur in Seitenansicht, im Querschnitt und in perspektivischer Ansicht.

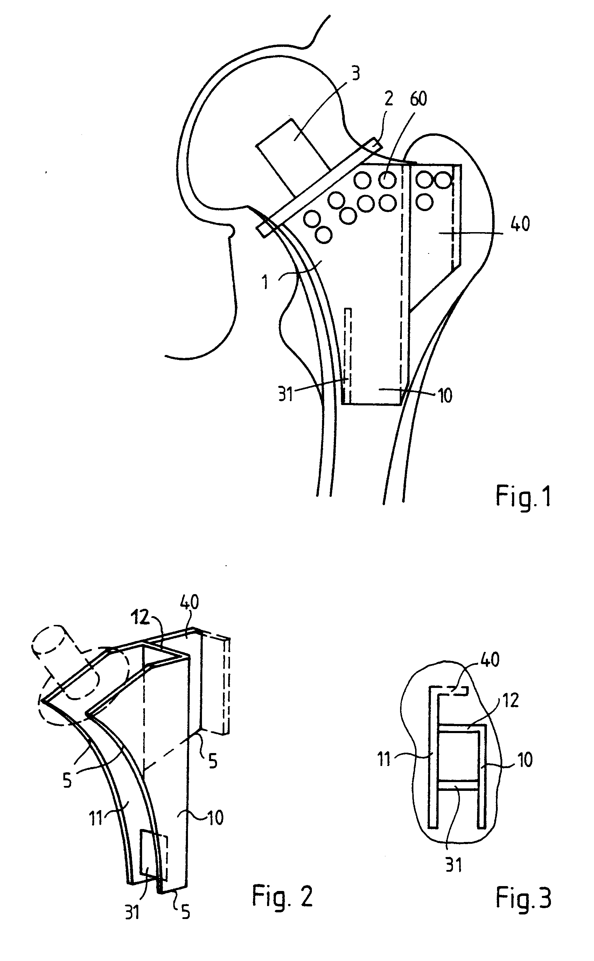

In den Figuren 1 bis 3 ist eine intertrochantäre Lamellenprothese in perspektivischer Ansicht, im Querschnitt und in Seitenansicht dargestellt, wobei die Seitenansicht schematisch in einen Femur eingezeichnet ist, um die Lage und die der Knochenform angepaßte Form der Endoprothese zu verdeutlichen.

Die Lamellenprothese besteht aus einem Prothesenschaft 1, einem Prothesenkragen 2 und einem konischen Zapfen 3 zur Aufnahme eines nicht näher dargestellten Prothesenkopfes. Der Prothesenschaft 1 weist drei parallel zur Längsachse des Prothesenschafts 1 gerichtete und plattenförmige ausgebildete Lamellen 10, 11, 12 auf, deren periphere Kanten als Schneidkanten 5 ausgebildet sind. Eine der Schneidkanten 5 der Lamellen 10, 11 ist bogenförmig ausgebildet und damit der äußeren Form des Femur nachgebildet.

Die Lamelle 11 ist mit einem Verlängerungssteg 40 verbunden, wobei die Verbindung einstückig erfolgen kann. Auch der Verlängerungssteg 40 ist mit einer unteren Schneidkante 5 versehen.

Die beiden parallel zueinander angeordneten Lamellen 10, 11 sind über eine Verbindungslamelle 31 miteinander verbunden, die an ihrer Unterseite ebenfalls eine Schneidkante 5 aufweist.

Die Lamellen 10, 11, 12 und der Verlängerungssteg 40 ist mit Bohrungen 60 versehen, in die spongiöses Material einwachsen kann.

Die in den Figuren 1 bis 3 dargestellte, im Querschnitt U-förmige Lamellenprothese zeichnet sich durch eine große Oberfläche zum An- und Einwachsen spongiösen Materials und durch eine optimale, der Knochenform angepaßte Form aus. Durch die in Längsrichtung des Prothesenschaftes 1 parallel verlaufenden Lamellen 10, 11, 12, die Verbindungslamelle 31 und den Verlängerungssteg 40 kann die Lamellenprothese senkrecht zur Femurachse eingesteckt bzw. eingeschlagen werden, ohne daß ein Vormeißeln oder vorbohren notwendig ist.

Infolge der Schneidkanten 5 wird das Eintreiben der intertrochantären Lamellenprothese erleichtert. Auf diese Weise wird die normale Physiologie des Knochens voll erhalten und man hat bei einer eventuell notwendig werdenden Revisionsoperation noch ausreichend Knochensubstanz zur Verfügung, wobei die Resektion der Endoprothese bei einer Revisionsoperation wegen der parallel verlaufenden Teilflächen ohne wesentliche Beschädigung des Knochens erfolgt.

Die offene Strukturierung der in den Figuren 1 bis 3 dargestellten Endoprothese schafft die Voraussetzung dafür, daß die Prothese durch und durch mit Spongiosa durchwachsen werden kann, was ein Auslockern der Prothese auch bei starker und wechselnder Belastung verhindert.

Der lamellenförmige Aufbau der Prothese ermöglicht nicht nur eine individuelle Anpassung der Prothese an die jeweilige Anatomie des Knochens mit einfachen technischen Mitteln, da jeweils nur plattenförmige Schafteile zu bearbeiten sind, sondern schafft zusätzlich auch die Voraussetzung dafür, daß die Lamellen und/oder verbindungslamellen und/oder Verlängerungsstege so geformt werden können, daß unter Berücksichtigung der jeweiligen Anatomie des Knochens eine optimale Krafteinleitung und gleichmäßige Kraftverteilung ohne Druckkonzentrationen erfolgen kann.

3

Durch zusätzliche Verwendung eines bioverträglichen Materials mit Mikroporösität für die Lamellen und Verbindungsstege kann die Oberflächenstruktur weiter verbessert werden, so daß die Festigkeit des Sitzes der Endoprothese im Knochen erhöht wird.

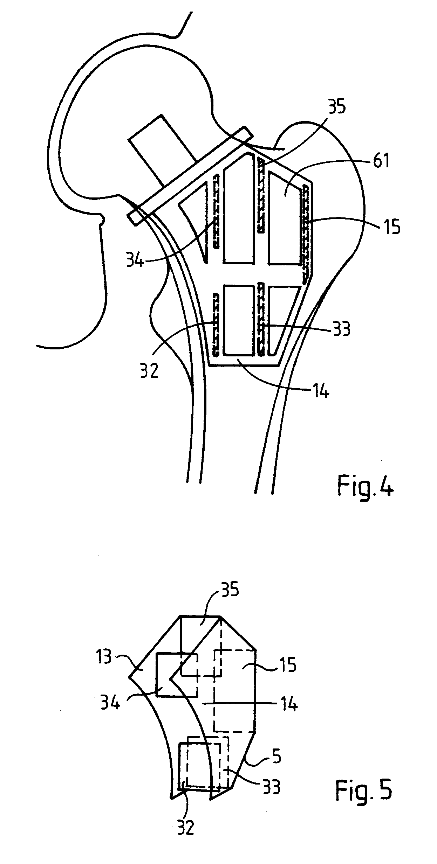

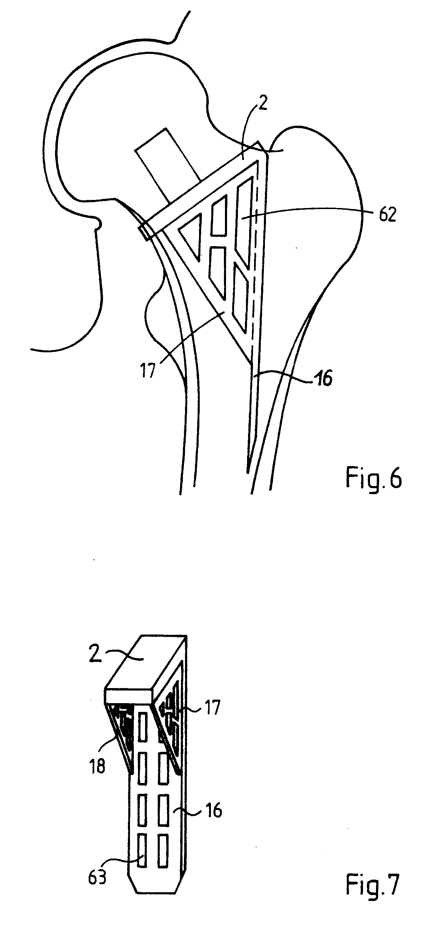

In den Figuren 4 und 5 ist eine Variante der intertrochantären Lamellenprothese dargestellt, die aus zwei parallel zueinander angeordneten Hauptlamellen 13, 14 und einer die parallelen Hauptlamellen 13, 14 verbindenden Lamelle 15 besteht. Zusätzlich sind die beiden Hauptlamellen 13, 14 durch verbindungslamellen 32 bis 35 miteinander verbunden, die zur vergrößerung der Oberfläche beitragen. Auch hier weisen die Lamellen periphere Schneidkanten 5 auf. Die Hauptlamellen 13, 14, 15 und ggf. die verbindungslamellen 32 bis 35 sind mit Durchbrüchen 61 versehen, die das Durchwachsen spongiösen Materials ermöglichen.

Wie insbesondere der Seitenansicht gemäß Figur 4 zu entnehmen ist, ist diese Prothesenform der Knochenform optimal angepaßt, so daß eine maximale Oberfläche genutzt wird.

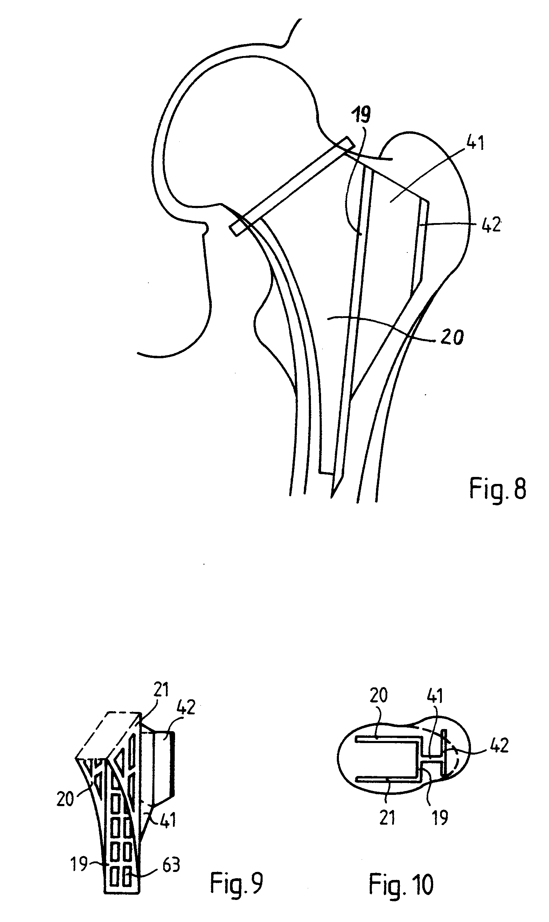

Figur 6 und 7 zeigt eine intertrochantäre Lamellenprothese mit einer plattenförmigen Hauptlamelle 16, die über zwei dreieckförmige seitliche Lamellen 17, 18 mit dem Prothesenkragen 2 verbunden ist. Die Hauptlamelle 16 ist an ihrem distalen Ende trapezförmig verjüngt ausgebildet und weist Durchbrüche 63 auf, die in dieser Ausführungsform rechteckförmig ausgebildet sind. Die in den dreieckförmigen Seitenlamellen 17, 18 vorgesehenen Durchbrüche 62 sind der äußeren Form der Seitenlamellen 17, 18 angepaßt.

Die Seitenansicht gemäß Figur 6 verdeutlicht die untere Schneidkante der Hauptlamelle 16, die ein leichtes Eintreiben der intertrochantären Lamellenprothese in den Femur ermöglicht.

In den Figuren 8 bis 10 ist eine Variante der intertrochantären Lamellenprothese gemäß den Figuren 6 und 7 dargestellt.

Diese Prothese besteht ebenfalls aus einer Hauptlamelle 19 mit Durchbrüchen 63 und seitlich angesetzten Seitenlamellen 20, 21, deren periphere Schneidkanten bogenförmig der Knochenform angepaßt ausgebildet sind. Zusätzlich ist mittig an die Hauptlamelle 19 ein Verlängerungssteg 41 angesetzt, der rechtwinklig mit einer Verlängerungsplatte 42 verbunden ist, so daß eine T-förmige Verlängerung gegeben ist. Auch in dieser Ausführungsform sind die peripheren Kanten als Schneidkanten ausgebildet.

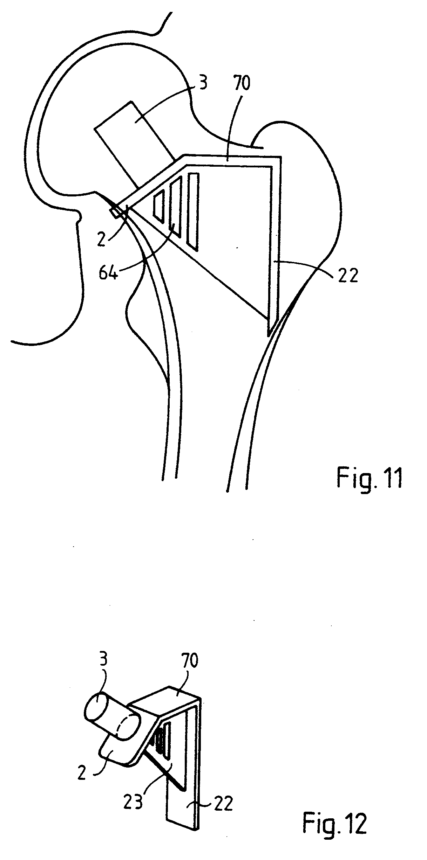

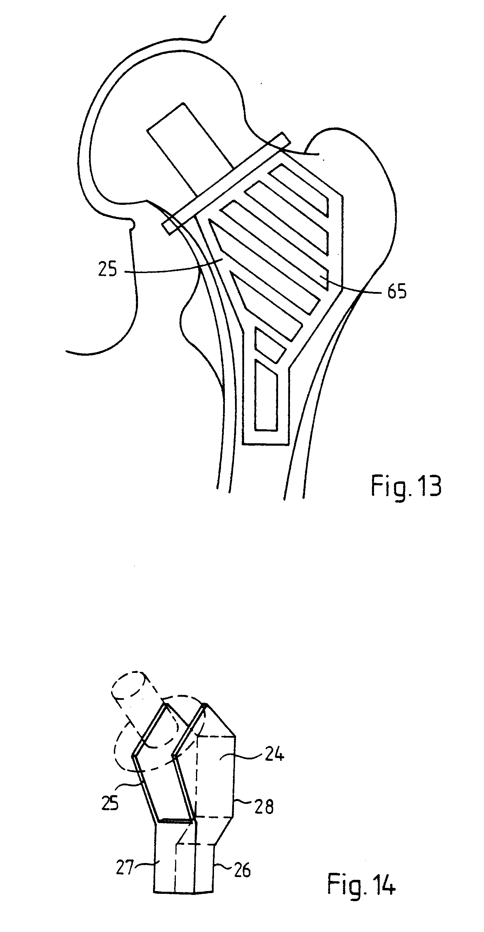

Die Figuren 11 und 12 zeigen in Seitenansicht bzw. in perspektivischer Ansicht eine intertrochantäre Lamellenprothese mit einer Hauptlamelle 22, die etwa im rechten Winkel mit einer oberen Deckfläche (Lamelle 70) verbunden ist, an die sich der Prothesenkragen 2 mit dem konisch geformten Zapfen 3 zur Aufnahme des Prothesenkopfes anschließt. Mittig auf die Hauptlamelle 22 aufgesetzt ist eine Lamelle 23, deren äußere Kanten mit der Deckfläche (Lamelle 70) und dem Prothesenkragen 2 verbunden sind. Die untere periphere Kante der Lamelle 23 ist ebenso wie die Unterkante der Hauptlamelle 22 als Schneidkante ausgebildet.

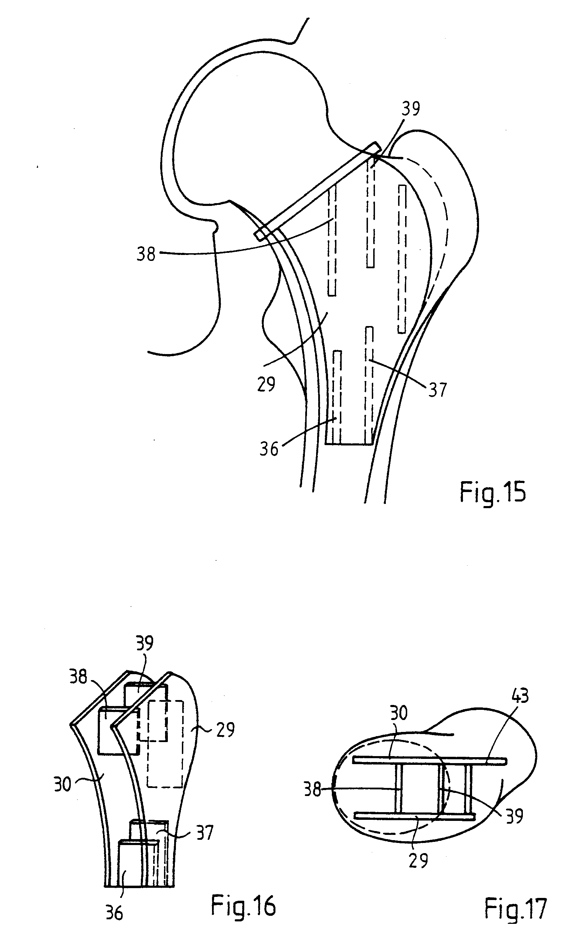

In den Figuren 13 und 14 ist in Seitenansicht bzw. in perspektivischer Ansicht eine kasten- oder trichterförmige intertrochantäre Lamellenprothese dargestellt. Sie besteht aus parallel zueinander verlaufenden Lamellen 24, 25 sowie die beiden Lamellen 24, 25 miteinander verbindenden, parallel zur Längsrichtung des Prothesenschaftes ausgerichtete Lamellen 26, 27, 28. Auch in dieser Ausführungsform sind die jeweils peripheren Kanten der Lamellen 24 bis 28 als Schneidkanten ausgebildet und mit Durchbrüchen 65 versehen, die der äußeren Geometrie der Lamellen angepaßt sind.

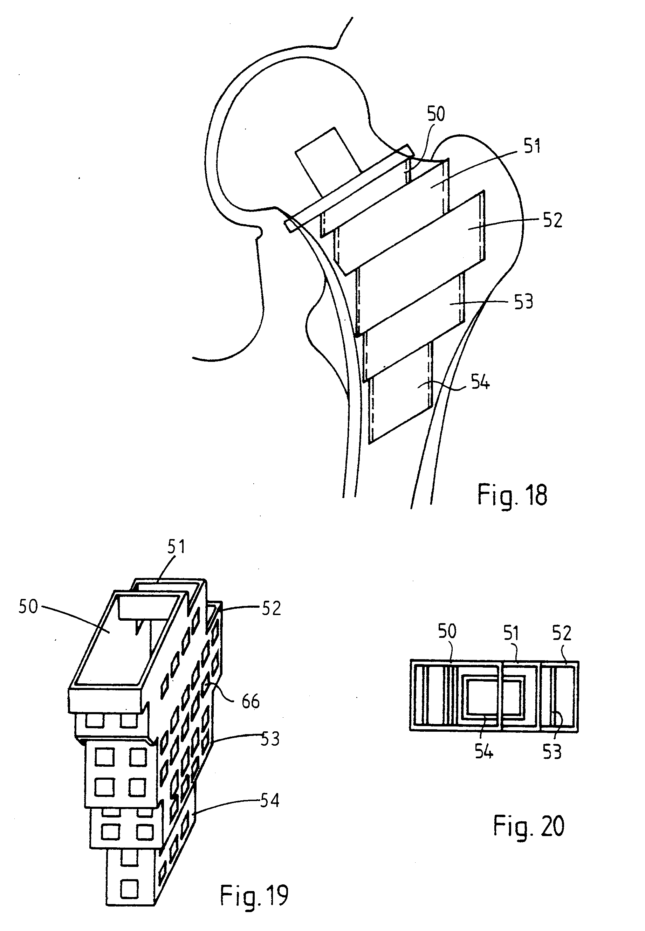

In den Figuren 15 bis 17 ist in Seitenansicht, perspektivischer Ansicht und im Querschnitt eine kasten- oder trichterförmige intertrochantäre Lamellenprothese dargestellt, die aus zwei parallel zueinander verlaufenden ventralen und dorsalen Lamellen 29, 30 mit dazwischen angeordneten, beide Lamellen 29, 30 miteinander verbindenden Verbindungslamellen 36 bis 39 besteht. Wie der Querschnittsdarstellung gemäß Figur 17 zu entnehmen ist, weist die eine Lamelle 30 einen verlängerten Steg 43 auf, der im implantierten Zustand im großen Rollhügel angeordnet ist. In den Figuren 18 bis 20 ist eine intertrochantäre Lamellenprothesen mit im Querschnitt geschlossener Kontur dargestellt, wobei diese Darstellung nur eine der möglichen geschlossenen Konturen ist. Die einzelnen Lamellen der kastenförmig gestaffelten intertrochantären Lamellenprothese weisen eine im Querschnitt rechteckförmige geschlossene Struktur auf und die einzelnen Elemente 50 bis 54 sind versetzt zueinander und der äußeren Kontur des Knochens angepaßt miteinander verbunden. Die jeweils herausragenden unteren peripheren Kanten sind als Schneidkanten ausgebildet. Zusätzlich weisen die Elemente 50 bis 54 Durchbrüche 66 auf, die das Einwachsen der Spongiosa ermöglichen.

Die vorstehend beschriebenen intertrochantären Lamellenprothesen zeichnen sich dadurch aus, daß die Druckkraftübertragung bzw. -weiterleitung in den Knochen proximal vom Hüftkopf mit seiner Spongiosa und von den spongiösen und cortikalen Anteilen des Schenkelhalses erfolgt. Dabei wird bei der Anbringung der Prothese die Physiologie des Markraumes einschließlich der intertrochanären Spongiosa erhalten, so daß eine biologische Anpassung im Rahmen der Alterung möglich ist. Im Gegensatz zu bekannten Prothesentypen, bei denen die Druckkraft am Calcar Femoris um 60% reduziert wird, was zu einer röntgenologisch nachweisbaren Resorption führt, wird bei den vorstehend beschriebenen Lamellenprothesen die Kraft physiologisch eingeleitet, wobei ein sicherer Aufsitz der Prothesen am Schenkelhals und eine optimale Verankerung im proximalen Femur sichergestellt ist.

4

Die Zusammensetzung der Prothesen aus einzelnen Lamellen ermöglicht es, mit einfachen technischen Mitteln jede Oberschenkelhalskonfiguration zu berücksichtigen und die Prothese entsprechend anzupassen. Dabei können unterschiedliche Krümmungen des Schenkelhalses und verschiedene Raumgrößen der regio intertrochanterica hinreichend berücksichtigt werden.

Zusätzlich führt die Lamellenform der Endoprothesen zu der Möglichkeit individuell anfertigbarer Prothesen nach einer Röntgenschablone, wobei die jeweils seitlichen Lamellen nach Röntgenschablonen geschnitten werden können. Außerdem ist ein baukastenartiges individuelles Zusammensetzen der Endoprothesen möglich.

Die jeweils peripheren, in den Knochen einzuschlagenden Kanten der Lamellen sind als Schneidkanten ausgebildet, was zu einer extremen Vereinfachung der Operation führt und eine nahezu vollständige Erhaltung der natürlichen Knochensubstanz ermöglicht. Die Lamellen sind vorzugsweise aus Titanblech gefertigt, welches mit bekannten biokompatiblen Oberflächengestaltungen bzw. -beschichtungen versehen sein kann.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.


**Patentansprüche**

1.  Schaftendoprothese, insbesondere mit Kragen (2), mit als plattenförmige Lamellen (10 bis 39, 70) ausgebildeten Schaftteilen zur, vorzugsweise zementlosen, Verankerung, wobei die Flächen mindestens zweier Lamellen parallel zur Längsachse des Prothesenschafts (1) gerichtet sind, und diese Flächen selbst derart ausgerichtet sind, daß ihre Flächenvektoren aufeinander senkrecht stehen, **dadurch gekennzeichnet,** daß mindestens eine zusätzliche plattenförmige Lamelle vorgesehen ist, und daß die Lamellen (10 bis 39, 70) derart angeordnet sind, daß eine Lamelle zwei Lamellen miteinander verbindet.

2.  Schaftendoprothese nach einem der vorangehenden Ansprüche, **dadurch gekennzeichnet** , daß mindestens zwei Lamellen (10 bis 39, 70) winklig aneinandergrenzen und einen räumlichen Kantenbereich bilden.

3.  Schaftendoprothese nach Anspruch 2 , **dadurch gekennzeichnet** , daß mindestens zwei Lamellen (10 bis 39, 70) rechtwinklig aneinandergrenzen.

4.  Schaftendoprothese nach einem der vorangehenden Ansprüche, **dadurch gekennzeichnet** , daß die Lamellen (10 bis 39) in Einschlagrichtung Kanten aufweisen, die als Schneidkanten (5) ausgebildet sind.

5.  Schaftendoprothese nach einem der vorangehenden Ansprüche, **dadurch gekennzeichnet** , daß die Lamellen (10 bis 39, 70) winkel- oder U-förmig miteinander verbunden sind.

6.  Schaftendoprothese nach Anspruch 1, **dadurch gekennzeichnet** , daß die Lamellen (10 bis 39, 70) im Querschnitt dreieck-, rechteck-, trapez- oder vieleckförmig miteinander verbunden sind.

7.  Schaftendoprothese nach einem der vorangehenden Ansprüche, **dadurch gekennzeichnet** , daß die Außenkontur der Lamellen (10 bis 39, 70) der Kontur des Knochens zur Aufnahme des Prothesenschaftes (1) angepaßt ist.

8.  Schaftendoprothese nach einem der vorangehenden Ansprüche, **dadurch gekennzeichnet** , daß mehrere plattenförmige Lamellen (31 bis 39), die Lamellen (10 bis 30) miteinander verbinden, gegeneinander in Längsrichtung des Prothesenschaftes (1) versetzt angeordnet sind.

9.  Schaftendoprothese nach einem der vorangehenden Ansprüche **dadurch gekennzeichnet** , daß einige der plattenförmigen Lamellen (31 bis 39), die Lamellen (10 bis 30) miteinander verbinden, in Längsrichtung des Prothesenschaftes (1) relativ zu anderen in gedrehter Position angeordnet sind.

10. Schaftendoprothese nach einem der vorangehenden Ansprüche, **gekennzeichnet durch** mit den Lamellen (10 bis 30) verbundenen Verlängerungsstegen (40 bis 43).

**11.** Schaftendoprothese nach einem der vorangehenden Ansprüche, **dadurch gekennzeichnet** , daß mehrere zu einer im Querschnitt offenen oder geschlossenen Kontur zusammengesetzte Lamellen (10 bis 30) als Elemente (50 bis 54) in Längsrichtung des Prothesenschaftes (1) miteinander verbunden und gegeneinander versetzt angeordnet sind.

**12.** Schaftendoprothese nach einem der vorangehenden Ansprüche, **dadurch gekennzeichnet** , daß die Lamellen (10 bis 39, 70) und/oder Verlängerungsstege (40 bis 43) Bohrungen und/oder Durchbrüche (60 bis 66) aufweisen.

**13.** Schaftendoprothese nach einem der vorangehenden Ansprüche, **dadurch gekennzeichnet** , daß die Lamellen (10 bis 39, 70) und/oder Verlängerungsstege (40 bis 43) aus einer stabilen Drahtgitterkonstruktion bestehen.

**14.** Schaftendoprothese nach einem der vorangehenden Ansprüche, **dadurch gekennzeichnet** , daß die Lamellen (10 bis 39, 70) und/oder Verlängerungsstege (40 bis 43) aus einem mikroporösen, bioverträglichen Material bestehen.

**15.** Verfahren zur Herstellung einer Schaftendoprothese nach einem der vorangehenden Ansprüche, **dadurch gekennzeichnet** , daß die ventralen und dorsalen Lamellen (10 bis 30) nach einer von dem mit der Prothese zu versehenden Knochen eines Patienten angefertigten Röntgenschablone geschnitten und die einzelnen Lamellen (10 bis 30) individuell der Form des Schenkelhalses bzw. des Trochantermassivs angepaßt zusammengesetzt werden.

## Claims

1) Shaft-type endoprosthesis, more particlarly with a collar (2) and with shaft portions for, preferably cement-free, anchorage in the form of plate-shaped fins (10 to 39, 70), the surfaces of at least two fins being arranged parallel to the longitudinal axis of the shaft (1) of the prosthesis, and the surfaces themselves being aligned so that their surface vectors are perpendicular to one another, characterised in that at least one additional plate-shaped fin is provided, and in that the fins (10 to 39, 70) are arranged so that one fin connects two fins to one another.

2) Shaft-type endoprosthesis according to one of the preceding claims, characterised in that at least two fins (10 to 39, 70) are contiguous at an angle to one another and form a three-dimensional edge portion.

3) Shaft-type endoprosthesis according to claim 2, characterised in that at least two fins (10 to 39, 70) are contiguous at right angles to each other.

4) Shaft-type endoprosthesis according to one of the preceding claims, characterised in that the fins (10 to 39), in the direction of insertion, have edges in the form of cutting edges (5).

5) Shaft-type endoprosthesis according to one of the preceding claims, characterised in that the fins (10 to 39, 70) are connected to one another in an angular or U-shaped configuration.

6) Shaft-type endoprosthesis according to claim 1, characterised in that the fins (10 to 39, 70) are connected to one another to form a triangle, rectangle, trapezoid or polygon in cross-section.

7) Shaft-type endoprosthesis according to one of the preceding claims, characterised in that the outer contour of the fin (10 to 39, 70) is adapted to fit the contour of the bone which is to receive the shaft (1) of the prosthesis.

8) Shaft-type endoprosthesis according to one of the preceding claims, characterised in that a plurality of plate-shaped fins (31 to 39) which join the fins (10 to 30) together, are arranged opposite and offset from one another in the longitudinal direction of the shaft (1) of the prosthesis.

9) Shaft-type endoprosthesis according to one of the preceding claims characterised in that some of the plate-shaped fins (31 to 39) which connect the fins (10 to 30) to one another, are arranged in a rotated position relative to others in the longitudinal direction of the shaft (1) of the prosthesis.

10) Shaft-type endoprosthesis according to one of the preceding claims, characterised by extension pins (40 to 43) connected to the fins (10 to 30).

11) Shaft-type endoprosthesis according to one of the preceding claims, characterised in that a plurality of fins (10 to 30) assembled to form a contour which is open or closed in cross-section are interconnected as elements (50 to 54) in the longitudinal direction of the shaft (1) of the prosthesis and are arranged opposite and offset from one another.

12) Shaft-type endoprosthesis according to one of the preceding claims, characterised in that the fins (10 to 39, 70) and/or extension pins (40 to 43) have bore holes and/or openings (60 to 66).

13) Shaft-type endoprosthesis according to one of the preceding claims, characterised in that the fins (10 to 39, 70) and/or extension pins (40 to 43) consist of a stable wire mesh construction.

14) Shaft-type endoprosthesis according to one of the preceding claims, characterised in that the fins (10 to 39, 70) and/or extension pins (40 to 43) consist of a microporous, biocompatible material.

15) A process for producing a shaft-type endoprosthesis according to one of the preceding claims, characterised in that the ventral and dorsal fins (10 to 30) are cut according to an X-ray template prepared from the patient's bone which is to be fitted with the prothesis and the individual fins (10 to 30), individually adapted to the shape of the neck of the femur or the body of the trochanter, are assembled.

**Revendications**

1. Endoprothèse à tige, notamment avec collerette (2), comportant des parties de tige constituées par des lamelles (10 à 39, 70) en forme de plaque, destinée de préférence à l'ancrage sans ciment, dans laquelle les surfaces d'au moins deux lamelles sont orientées parallèlement à l'axe longitudinal de la tige (1) de la prothèse, et ces surfaces sont elles-mêmes orientées de façon que leurs vecteurs de surface soient perpendiculaires entre eux, caractérisée

   en ce qu'il est prévu au moins une lamelle en forme de plaque supplémentaire et en ce que les lamelles (10 à 39, 70) sont disposées de manière qu'une lamelle assemble deux lamelles l'une à l'autre.

2. Endoprothèse à tige selon une des revendications précédentes, caractérisée en ce qu'au moins deux lamelles (10 à 39, 70) se rejoignent en formant un angle et forment une région d'arête tridimensionnelle.

3. Endoprothèse à tige selon la revendication 2, caractérisée en ce qu'au moins deux lamelles (10 à 39, 70) se rejoignent à angle droit.

4. Endoprothèse à tige selon une des revendications précédentes, caractérisée en ce que les lamelles (10 à 39) présentent des bords orientés selon la direction de l'enfoncement.

5. Endoprothèse à tige selon une des revendications précédentes, caractérisée en ce que les lamelles (10 à 39, 70) sont assemblées les unes aux autres en formant un angle ou en formant un U.

6. Endoprothèse à tige selon la revendication 1, caractérisée en ce que les lamelles (10 à 39, 70) sont réunies les unes aux autres en formant en section transversale un triangle, un rectangle, un trapèze ou un polygone.

7. Endoprothèse à tige selon une des revendications précédentes, caractérisée en ce que le contour extérieur des lamelles (10 à 39, 70) est adapté au contour de l'os destiné à recevoir la tige (1) de la prothèse.

8. Endoprothèse à tige selon une des revendications précédentes, caractérisée en ce que plusieurs lamelles en forme de plaque (31 à 39) qui assemblent les lamelles (10 à 30) les unes aux autres sont décalées l'une par rapport à l'autre selon la direction longitudinale de la tige (1) de la prothèse.

9. Endoprothèse à tige selon une des revendications précédentes, caractérisée en ce que certaines des lamelles en forme de plaque (31 à 39) qui assemblent les lamelles (10 à 30) les unes aux autres sont disposées dans des positions tournées les unes par rapport aux autres dans la direction longitudinale de la tige (1) de la prothèse.

10. Endoprothèse à tige selon une des revendications précédentes, caractérisée par des nervures prolongatrices (40 à 43) reliées aux lamelles (10 à 30).

11. Endoprothèse à tige selon une des revendications précédentes, caractérisée en ce que plusieurs lamelles (10 à 30) qui sont assemblées pour former un contour ouvert ou fermé en section transversale forment des éléments (50 à 54) qui sont assemblés les uns aux autres dans la direction longitudinale de la tige (1) de la prothèse et sont décalés les uns par rapport aux autres.

12. Endoprothèse à tige selon une des revendications précédentes, caractérisée en ce que les lamelles (10 à 39, 70) et/ou les nervures prolongatrices (40 à 43) présentent des perçages et/ou ajours (60 à 66).

13. Endoprothèse à tige selon une des revendications précédentes, caractérisée en ce que les lamelles (10 à 39, 70) et/ou les nervures prolongatrices (40 à 43) sont composées d'une construction solide, en grillage de fils métalliques.

14. Endoprothèse à tige selon une des revendications précédentes, caractérisée en ce que les lamelles (10 à 39, 70) et/ou les nervures prolongatrices (40 à 43) sont composées d'une matière microporeuse physiologiquement compatible.

15. Procédé de fabrication d'une endoprothèse à tige selon une des revendications précédentes, caractérisé en ce que les lamelles ventrales et dorsales (10 à 30) sont découpées selon un gabarit radiographique préparé en fonction de l'os du patient qu'il s'agit de munir de la prothèse et les différentes lamelles (10 à 30) sont assemblées dans une disposition adaptée individuellement à la forme du col du fémur ou du massif trochantérien.

Fig.1

Fig. 2

Fig.3

Fig.4

Fig.5

Fig. 6

Fig. 7

Fig. 8

Fig. 9

Fig. 10

Fig. 11

Fig. 12

25    65

Fig. 13

24
25
28
27    26

Fig. 14

Fig.15

Fig.16

Fig.17

Fig. 18

Fig. 19

Fig. 20